# EUROPEAN PATENT SPECIFICATION

(11) **EP 0 569 393 B1**
(45) Date of publication and mention of the grant of the patent: **18.10.1995**
(21) Application number: 92902884.3
(22) Date of filing: 28.01.1992
(51) Int. Cl.: C07C 233/36, A61K 31/16, C07C 233/47

(54) **DERIVATIVES OF DICARBOXYLIC ACIDS HAVING SIX TO TWELVE CARBON ATOMS AND USE OF THESE DERIVATIVES AND DICARBOXYLIC ACIDS ALONE IN THE PREPARATION OF PHARMACEUTICAL COMPOSITIONS FOR ENTERAL AND PARENTERAL NUTRITION**
DICARBONSÄURE-DERIVATE VON 6-12 KOHLENSTOFFATOMEN SOWIE ANWENDUNG DIESER DERIVATE SOWIE DER FREIEN DICARBONSÄUREN ZUR HERSTELLUNG VON ARZNEIMITTELN ZUR ENTERALEN UND PARENTERALEN ERNÄHRUNG
DERIVES D'ACIDES DICARBOXYLIQUES POSSEDANT DE SIX A DOUZE ATOMES DE CARBONE, ET UTILISATION DE CES DERIVES ET DES ACIDES DICARBOXYLIQUES SEULS POUR LA PREPARATION DE COMPOSITIONS PHARMACEUTIQUES DESTINEES A LA NUTRITION ENTERALE ET PARENTERALE

(30) Priority: 29.01.1991 IT RM91000
(43) Date of publication of application: 18.11.1993
(73) Proprietor: PINAROO LTD., Dublin 2 (IE)
(72) Inventor: CASTAGNETO, Marco, I-00168 Roma (IT)
(74) Representative: Cavattoni, Fabio
(86) International application number: IT9200006
(87) International publication number: WO9212960

(56) References cited:
- DE-C- 854 357
- US-A- 4 999 417
- Chemical Abstracts, vol. 113, no. 13, 24 September 1990 (Columbus, Ohio, US) K.Y. Tserng et al.: "Abnormal urinary excretion of unsaturated dicarboxylic acids in patients with medium-chain acyl-CoA dehydrogenase deficiency", see page 500,abstract 113242a, & J. Lipid. Res. 1990, 31(5), 763-71
- Chemical Abstracts, vol. 91, no. 5, 30 July 1979 (Columbus, Ohio, US) R.J.W.Truscott et al.: "Dicarboxylic aciduria: the response to fasting", see page 432, abstract 37005k, & Clin. Chim. Acta, 1979, 94(1), 31-9
- Chemical Abstracts, vol. 85, no. 13, 27 September 1976 (Columbus, Ohio, US)N.Gregersen et al.: "Suberylglycine excretion in the urine from a patient with dicarboxylic aciduria", see page 418, abstract 91624a, & Clin. Chim. Acta,1976, 70(3), 417-25
- Chemical Abstracts, vol. 88, no. 19, 8 May 1978 (Columbus, Ohio, US) I. Groenet al.: "Formation of N-dicarboxyl-mono-glycines catalyzed by glycine-N-acylase", see page 185, abstract 132529f, & Biochem. Med. 1978, 19(1), 133-40
- Chemical Abstracts, vol. 65, no. 4, 15 August 1966 (Columbus, Ohio, US) H. Zahn et al.: "Oligomers. XLIV. Propionyl derivatives and propyl amides of linear nylon 66 oligomers", see column 5360g/h, & Kolloid-Z. Z. Polymere 208(2), 132-7(1966)
- Chemical Abstracts, vol. 61, no. 4, 17 August 1964 (Columbus, Ohio, US) W.Flitsch: "Preparation of cyclic imides", see column 4310g/h, & Ber. 97(6),1542-7(1964)
- Chemical Abstracts, vol. 55, no. 21, 16 October 1961 (Columbus, Ohio, US) L.Friedman et al.: "Amidation and amino alkylation of olefins. Blocking techniques in radical chain reactions", see column 20934e/f/g/h/i, & Tetrahedron Letters 1961, 238-42
- Chemical Abstracts, vol. 60, no. 3, 17 February 1964 (Columbus, Ohio, US) N.Wada et al.: "Rust preventive effect of dibasic acid alkylamides", see column 4209f/g/h, & Osaka Furitsu Kogyo-Shoreikan Hokoku, no. 26, 58-62(1961)

## Description

This invention relates to new dicarboxylic acid derivatives having 6 to 12 carbon atoms and the use of both C6-C12 dicarboxylic acids and the new derivatives thereof in the preparation of pharmaceutical compositions for enteral and parenteral nutrition.

Sebacic acid (C10), pharmacologically acceptable salts of sebacic acid, the triglyceride of sebacic acid and pharmacologically acceptable salts of the triglyceride of sebacic acid are to be considered excluded from C6-C12 dicarboxylic acids and derivatives thereof. Accordingly, even if in the course of this description C6 - C12 dicarboxylic acids and their derivatives are referred to for the sake of simplicity, sebacic acid and its above derivatives are intended to be excluded.

The introduction of lipids to total parenteral nutrition (TPN) has represented an important clinical breakthrough in the past 20 years. Their principal advantages are due to the fact that a) the emulsions used are isoosmolar with plasma and can therefore be administered through a peripheral vein; b) long chain triglycerides (LCT) possess a caloric equivalent which is significantly higher than that of glucose (9 kcal/g); c) the oxidation of the fatty acids is characterized by a respiratory quotient (RQ) of 0.7, and thus is associated with a low production of CO₂, which is of course important in patients with pulmonary distress, and d) their metabolism is not insulin-dependent.

However, their principal disadvantage is connected with the possibility of a reduced clearance of plasma triglycerides due to the altered function of the lipo-protein lipasis enzyme (LPL) and consequent microembolisms at the cerebral and pulmonary capillary level. Also the resulting hyperlipemia brings about a depression of the immunological and granulocyte response and sometimes intra-hepatic cholestasis.

In some pathological conditions where there is a reduced tissue utilization of conventional substrates [glucose and long chain triglycerides (LCT)], such as decompensed diabetes mellitus, hyperlipidemia, states of incomplete enzymatic maturity as in premature newborns, states of hypercatabolism due to stress, trauma, burns and more advanced phases of the septic process, the introduction of new substrates may enable better results to be obtained in terms of effective energy yield and correction of the primitive metabolic block.

For these reasons, medium chain triglycerides (MCT) containing medium chain mono-carboxylic fatty acids (MCFA) of 6-12 carbon atoms have been proposed and introduced in TPN. Unlike LCT which are hydrolyzed by LPL, MCT are hydrolyzed in the liver to glycerol and medium chain fatty acids (MCFA). A small part of MCFA is re-esterified to triglycerides, while 80% undergoes β-oxidation and their passage across the mitochondrial membrane occurs irrespective of the presence of carnitine. However, the rapid β-oxidation of MCFA brings about an accumulation of acetyl-CoA with the consequent formation of ketone bodies which can cause e.g. ketoacidosis.

DE-C-854 357 discloses N-metyladipamic acid (CH₃NHCO(CH₂)₄COOH). US-A-4.999.417 discloses sebacyl (-CO(CH₂)₈CO-) and adipyl mono glycine. Chemical abstracts Vol. 113 (1990), abstract 113242a; chemical abstracts Vol. 91 (1979), abstract 37005k and chemical abstracts Vol. 85 (1976), abstract 91264a disclose suberylglycine. Chemical abstracts Vol. 88 (1978), abstract 132529f discloses mono glycine of adipic and suberic (HOOC(CH₂)₆COOH) acids. Chemical abstracts Vol. 65 (1966), abstract 5360g discloses various amides of adipic acid. Chemical abstracts Vol. 61 (1964), abstract 4310g discloses: 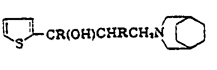
Chemical abstracts Vol. 55 (1961), abstract 20934d discloses Me₂CNHCO(CH₂)₁₀CO₂Me. Chemical abstracts Vol. 60 (1964), abstract 4209f discloses various amides of adipic and sebacic acids.

It has now been surprisingly found that medium chain dicarboxylic acids of 6 to 12 carbon atoms and derivatives thereof as hereinafter defined constitute an excellent substrate for the formulation of compositions to be used in enteral and parenteral nutrition while not having the disadvantages of monocarboxylic acids of the same number of carbon atoms.

Medium chain (C6-C12) dicarboxylic acids of the invention are linear chain acids with 2 carboxylic groups on the terminal position. These acids have not shown any toxic or teratogenic effect on laboratory animals. In experimental animals and man these diacids are β-oxidized, the chain being shortened by two carbon units each time until malonic acid (for odd-chain dicarboxylic acids) or succinic acid (for even-chain dicarboxylic acids) is achieved. The β-oxidation, which uses the same enzymatic pathways as oxidation in monocarboxylic fatty acids, takes place both in the mitochondria via a carnitine independent mechanism, and in peroxisomes.

Accordingly, an object of the present invention is to provide new linear chain derivatives of dicarboxylic acids with 6 to 12 carbon atoms; in particular:
a) amides of the above acids with aminoacids chosen from a group including glycine, alanine, valine, leucine, isoleucine, proline, lysine, arginine, asparagine, glutamine, cysteine, methionine, serine and threonine, hydroxyproline, or with aliphatic monoamine or diamine compounds and pharmacologically acceptable salts thereof;
b) esters of the above acids with serine, threonine and hydroxyproline (with the formation of the esters between serine, threonine and hydroxyproline hydroxylic group (-OH) and one of the carboxylic groups of said acids) and pharmacologically acceptable salts thereof;
c) esters of the above acids with L-carnitine and/or amino-acidic bond between the amino group present in the carnitine molecule and the other carboxylic group of dicarboxylic acids and pharmacologically acceptable salts thereof;
d) esters of the above acids with biliary acids (cholic acid, deoxycholic acid and lithocholic acid) either in free form or tauro- or glyco-conjugated, and pharmacologically acceptable salts thereof;
e) esters of the above acids with primary, secondary and tertiary aliphatic alcohols and pharmacologically acceptable salts thereof;
f) triglycerides of the above acids and pharmacologically acceptable salts thereof with the exclusion of the triglyceride of sebacic acid and pharmacologically acceptable salts thereof;
Preferably, the pharmacologically acceptable salts of the compounds (a) to (f) are sodium and potassium salts.

Another object of the present invention is the use of the above compounds (a) to (f), the dicarboxylic acids themselves and pharmacologically acceptable salts thereof (with the exception of sebacic acid and its pharmacologically acceptable salts) for producing pharmaceutical compositions suitable for enteral and parenteral nutrition.

A further object of the present invention is the use of the same compounds (a) to (f) and the above acids and pharmacologically acceptable salts thereof for producing a composition which can be administered orally and which is used as a dietetic or energetic integrator.

Another object of the present invention is the use of the same components (a) to (f) and the above acids and pharmacologically acceptable salts thereof for producing a pharmaceutical composition which can be administered orally or parenterally to improve nerve transmission and/or muscular motility.

One particular use of the esters (d) is for preparing pharmaceutical compositions to be administered orally to dissolve gallbladder or choledocic stones.

Accordingly, an object of the present invention is also to provide pharmaceutical compositions administered orally or parenterally comprising, as the active substance, an amount effective for reaching the therapeutic goal of at least one of the compounds (a) to (f) or of the above dicarboxylic acids or of pharmacologically acceptable salts thereof.

These compounds, when prepared in water solution, range from 0.2 to 1 mole/L of the compound (a) to (f) or of a dicarboxylic acid or of a pharmacologically acceptable salt of dicarboxylic acid, as previously described.

Compositions suitable for oral administration, in unit dosage form, comprise from 0.5 to 25 g of salts or derivatives of dicarboxylic acids, and can be used as energetic integrators in both normal or pathological conditions such as obesity, hyperlipidemia, atherosclerosis and ageing. In these pathologic conditions (and particularly in obesity) it is possible to use these substances as drugs.

Salts of the above dicarboxylic acids are obtained by reaction of the diacids with NaOH or KOH using 2 molar amounts of the basis.

In order to prepare the sodic or potassic salts of the mono-esters of the above dicarboxylic acids, it is necessary to previously synthetize the acid chlorides of dicarboxylic acids by adding thionyl chloride (bp 79°C) dissolved in freshly distilled dioxane on litium or aluminium hydroxyde (molar ratio dicarboxylic acid:thionyl chloride 1:2, w/w) and boiling the mixture for 4-8 hours using caesium chloride as catalyst. By removing dioxane under vacuum, the acid chloride of the dicarboxilic acid involved is obtained.

The synthesis of the esters of dicarboxylic acids is obtained by the Schotten-Baumann method which consists in shaking the acid chloride with acqueous sodium hydroxyde containing the appropriate alcohol, in order to maintain the pH of the solution around 8. At the end of the reaction, the ester is free from acid chloride and hydrogen chloride and can be extracted and dried immediately. The reaction is complete in about 30-60 minutes for small amounts of reagents. NaCl is then removed by dializing the solution against double distilled water over 24 hours.

Amides of dicarboxylic acids are synthetized by reacting in a basic medium an amine (primary, secondary or tertiary amine) with the acid chloride of the dycarboxylic acid. For example, in the case of a tertiary amine such as triethylamine:

ClCO-(CH₂)ₙ-COCl+3 R-NH₂+H₂O-->HOOC-(CH₂)ₙ-CONH-R+2 R-NH₃Cl

Dicarboxylic acid derivatives with carnitine, using the acid chloride of the dicarboxylic acid, have the following formula: 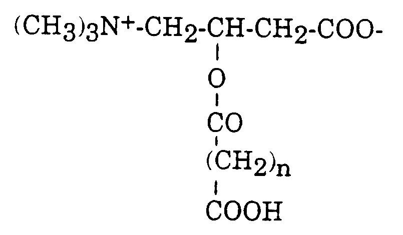
The salts of the above dicarboxylic acids or those of their derivatives are not toxic in laboratory animals, at least as far as the acute toxicity through the oral or i.p. route is concerned; however, the toxicity depends on the amounts of sodium or potassium administered.

Dicarboxylic acid derivatives with aminoacids, carnitine and glycerol (dicarboxylic acid triglycerides) can be employed in parenteral nutrition as well as the sodium or potassium salt thereof. In fact, they show a low urinary loss and the amount of dicarboxylic acid excreted with urine appears to be inversely proportional to their chain length: the longer the chain, the smaller the urinary excretion (however, dicarboxylic acids with a chain longer than C12 are not soluble in water). In addition:
- dicarboxylic acids present a good tissue utilization since even numbered dicarboxylic acids are completely oxidized to H₂O and CO₂ and odd numbered dicarboxylic acids are β-oxidized down to malonil-CoA, which can not be further oxidized and represents the starter in the synthesis of fatty acids;
- dicarboxylic acids are transported into the mitochondria to be β-oxidized via a carnitine-independent pathway;
- dicarboxylic acids are quickly oxidized at the level of peroxisomes;
- dicarboxylic acids show a direct cellular biodisponibility since they do not require an hydrolitic step at the level of LPL (like LCT) or of liver (like MCT);
- as dicarboxylic acids are highly water soluble in the salt form, they can be directly administered through a peripheral vein and do not require complex and expensive industrial preparations.

## Claims

1. Amides of linear chain dicarboxylic acids having a number of carbon atoms ranging between 6 and 12 with:
(i) aminoacids selected from the group comprising glycine, alanine, valine, leucine, isoleucine, proline, lysine, arginine, asparagine, glutamine, cysteine, methionine, serine, threonine and hydroxyproline;
(ii) aliphatic mono- and di-amines;
and pharmacologically acceptable salts thereof,
prodded that, if the amide is a monoamide, the aminoacid is not glycine or alanine if the carboxylic acid is adipic, suberic or sebacic acid.

2. Eaters of C6-C12 linear chain dicarboxylic acids with serine, threonine, hydroxyproline, L-carnitine, cholic, deoxycholic or lithocholic acids, either free or tauro- or glyco-conjugated, aliphatic primary, secondary or tertiary alcohols and pharmacologically acceptable salts thereof.

3. Triglycerides of C6-C12 linear chain dicarboxylic acids and pharmacologically acceptable salts thereof, excluding the triglyceride of sebacic acid and pharmacologically acceptable salts thereof.

4. Compounds according to claims 1 to 3 in the form of sodium or potassium salts.

5. Use of C6-C12 linear chain dicarboxylic acids, with the exception of sebacic acid, and pharmacologically acceptable salts thereof for producing pharmaceutical compositions for enteral and parenteral nutrition.

6. Use of the compounds according to claims 1 to 4 for producing pharmaceutical compositions for enteral and parenteral nutrition.

7. A pharmaceutical composition suitable for enteral and parenteral administration comprising, as active substance, a C6-C12 linear chain dicarboxylic acid, with the exception of sebacic acid, or pharmacologically acceptable salt thereof.

8. A pharmaceutical composition suitable for enteral and parenteral administration comprising, as active substance, a compound according to claims 1 to 4.

9. A composition according to claim 7 or 8 in the form of a water solution comprising 0.2 to 1 mole/liter of active substance.

10. A composition according to claim 7 or 8, suitable for oral administration, in unit dosage form, comprising 0.5 to 25 g of active substance.

11. An orally administrable composition, in unit dosage form, comprising 0.5 to 25 g of one of the compounds claimed in claims 1 to 4 or a C6-C12 linear chain dicarboxylic acid or a pharmaceutically acceptable salt thereof, as dietetic integrator and/or energy integrator.

12. Use of the esters of C6-C12 linear chain dicarboxylic acids with cholic, deoxycholic or lithocholic acids, either free or tauro- or glyco-conjugated for producing an orally administrable pharmaceutical composition for dissolving biliary stones.

13. Use of one of the compounds of claims 1 to 4 or a C6-C12 linear chain dicarboxylic acid or a pharmacologically acceptable salt thereof for producing an orally and parenterally administrable pharmaceutical composition for enhancing the nervous transmission and/or the muscular motility.

## Patentansprüche

1. Amide geradkettiger Dicarbonsäuren mit einer Kohlenstoffatomzahl zwischen 6 und 12 mit
i) Aminosäuren, die unter Glycin, Alanin, Valin, Leucin, Isoleucin, Prolin, Lysin, Arginin, Asparagin, Glutamin, Cystein, Methionin, Serin, Threonin und Hydroxyprolin ausgewählt sind,
ii) aliphatischen Mono- und Diaminen;
und pharmakalogisch akzeptable Salze hiervon, wobei gilt, daß wenn das Amid ein Monoamid ist, die Aminosäure nicht Glycin oder Alanin ist, wenn die Carbonsäure Adipin-, Suberin- oder Sebacinsäure ist.

2. Ester geradkettiger C₆-C₁₂-Dicarbonsäuren mit Serin, Threonin, Hydroxyprolin, L-Carnitin, Cholin, Desoxycholin oder Lithocholinsäure entweder in freier oder tauro- oder glycokonjugierter Form, aliphatischen, primären, sekundären oder tertiären Alkoholen und pharmakologisch akzeptable Salze hiervon.

3. Triglyceride von geradkettigen C-₆-C₁₂-Dicarbonsäuren und pharmakologisch akzeptablen Salzen hiervon, ausschließlich des Triglycerids von Sebacinsäure und pharmakologisch akzeptablen Salzen hiervon.

4. Verbindung nach einem der Ansprüche 1 bis 3 in Form des Natrium- und Kaliumsalzes.

5. Verwendung einer geradkettigen C₆-C₁₂-Dicarbonsäure, ausgenommen Sebacinsäure, und von pharmakologisch akzeptablen Salzen hiervon zur Herstellung pharmazeutischer Zubereitungen zur enteralen und parenteralen Ernährung.

6. Verwendung der Verbindungen nach einem der Ansprüche 1 bis 4 zur Herstellung pharmazeutischer Zubereitungen zur enteralen und parenteralen Ernährung.

7. Pharmazeutische Zubereitung mit einer Eignung zur enteralen und parenteralen Verabreichung mit einer geradkettigen C₆-C₁₂-Dicarbonsäure, ausgenommen Sebacinsäure, oder einem pharmakologisch akzeptablen Salz hiervon als Wirkstoff.

8. Pharmazeutische Zubereitung mit Eignung zur enteralen und parenteralen Verabreichung mit einer Verbindung nach einem der Ansprüche 1 bis 4 als Wirkstoff.

9. Zubereitung nach Anspruch 7 oder 8 in Form einer wäßrigen Lösung mit 0,2 bis 1 mol/l Wirkstoff.

10. Zubereitung nach Anspruch 7 oder 8 mit einer Eignung zur oralen Verabreichung in Einheitsdosisform, die 0,5 bis 25 g Wirkstoff umfaßt.

11. Oral verabreichbare Zubereitung in Einheitsdosisform, die 0,5 bis 25 g einer der Verbindungen nach einem der Ansprüche 1 bis 4 oder einer geradkettigen C₆-C₁₂-Dicarbonsäure oder eines pharmakologisch akzeptablen Salzes hiervon umfaßt, als diätetischen Integrator und/oder Energieintegrator.

12. Verwendung der Ester geradkettiger C₆-C₁₂-Dicarbonsäuren mit Cholin-, Desoxycholin- oder Lithocholinsäure entweder in freier oder in tauro- oder glycokonjugierter Form zur Herstellung einer oral verabreichbaren pharmazeutischen Zubereitung zur Auflösung von Gallensteinen.

13. Verwendung einer der Verbindungen nach einem der Ansprüche 1 bis 4 oder einer geradkettigen C₆-C₁₂-Dicarbonsäure oder eines pharmakologisch akzeptablen Salzes hiervon zur Herstellung einer oral und parenteral verabreichbaren pharmazeutischen Zubereitung zur Verstärkung der Nerventransmission und/oder Muskelmotilität.

## Revendications

1. Amides d'acides dicarboxyliques à chaîne linéaire comportant de 6 à 12 atomes de carbone, avec :
(i) des aminoacides choisis parmi la glycine, l'alanine, la valine, la leucine, l'isoleucine, la proline, la lysine, l'arginine, l'asparagine, la glutamine, la cystéine, la méthionine, la sérine, la thréonine et l'hydroxyproline ;
(ii) des mono et des diamines aliphatiques ; et leurs sels pharmacologiquement acceptables, à condition que si l'amide est un monoamide, l'aminoacide ne soit pas la glycine ou l'alanine si l'acide carboxylique est l'acide adipique, subérique ou sébacique.

2. Esters d'acides dicarboxyliques à chaîne linéaire en C6 à C12, avec la serine, la thréonine, l'hydroxyproline, la L-carnitine, l'acide cholique, désoxycholique ou lithocholique, sous forme libre ou tauro- ou glyco-conjuguée, les alcools primaires, secondaires ou tertiaires aliphatiques et leurs sels pharmacologiquement acceptables.

3. Triglycérides dérivés d'acides dicarboxyliques à chaîne linéaire en C6 à C12 et leurs sels pharmacologiquement acceptables, à l'exclusion du triglycéride de l'acide sébacique et de ses sels pharmacologiquement acceptables.

4. Composés selon les revendications 1 à 3, sous la forme de sels de sodium ou de potassium.

5. Utilisation d'acides dicarboxyliques à chaîne linéaire en C6 à C12, à l'exception de l'acide sébacique, et de leurs sels pharmacologiquement acceptables pour la préparation de compositions pharmaceutiques destinées à la nutrition par voies entérale et parentérale.

6. Utilisation des composés selon les revendications 1 à 4, pour la préparation de compositions pharmaceutiques pour la nutrition par voies entérale et parentérale.

7. Composition pharmaceutique appropriée pour une administration par voies entérale et parentérale, comprenant en tant que substance active, un acide dicarboxylique à chaîne linéaire en C6 à C12, à l'exception de l'acide sébacique, ou un sel pharmacologiquement acceptable de celui-ci.

8. Composition pharmaceutique appropriée pour une administration par voies entérale et parentérale, comprenant comme substance active, un composé selon les revendications 1 à 4.

9. Composition selon la revendication 7 ou 8, sous la forme d'une solution aqueuse comprenant de 0,2 à 1 mole/litre de substance active.

10. Composition selon la revendication 7 ou 8, appropriée pour une administration par voie orale, sous forme pharmaceutique unitaire, comprenant de 0,5 à 25 g de substance active.

11. Composition administrable par voie orale sous forme pharmaceutique unitaire, comprenant de 0,5 à 25 g de l'un des composés revendiqués dans les revendications 1 à 4, ou d'un acide dicarboxylique à chaîne linéaire en C6 à C12 ou d'un sel pharmaceutiquement acceptable de celui-ci, comme intégrateur diététique et/ou intégrateur énergétique.

12. Utilisation des esters d'acide dicarboxylique à chaîne linéaire en C6 à C12, avec l'acide cholique, désoxycholique ou lithocholique, sous forme libre, ou tauro- ou glyco-conjuguée, pour préparer une composition pharmaceutique administrable par voie orale afin de dissoudre des calculs biliaires.

13. Utilisation de l'un des composés selon les revendications 1 à 4 ou d'un acide dicarboxylique à chaîne linéaire en C6 à C12, ou d'un sel pharmacologiquement acceptable de celui-ci, pour préparer une composition pharmaceutique administrable par voies orale et parentérale, destinée à stimuler la transmission nerveuse et/ou la motilité musculaire.
